**Europäisches Patentamt**

**European Patent Office**

**Office européen des brevets**

(19)

(11) Veröffentlichungsnummer: **0 327 926**

**A1**

# EUROPÄISCHE PATENTANMELDUNG

(12)

(21) Anmeldenummer: **89101617.2**

(22) Anmeldetag: **31.01.89**

(51) Int. Cl.⁴: **C07C 143/10 , C07C 139/14**

(30) Priorität: **11.02.88 DE 3804149**

(43) Veröffentlichungstag der Anmeldung:
**16.08.89 Patentblatt 89/33**

(84) Benannte Vertragsstaaten:
**BE CH DE ES FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Reinhardt, Gerd, Dr.**
**Freiherr-vom-Stein-Strasse 37**
**D-6233 Kelkheim (Taunus)(DE)**

(54) **Verfahren zur Herstellung von Salzen von Acyloxialkansulfonsäuren.**

(57) Verfahren zur Herstellung von Acyloxialkansulfonaten der Formel

$$R-\overset{\overset{\displaystyle O}{\|}}{C}-O-A-SO_3M$$

wobei R $C_5$-$C_{21}$-Alkyl oder $C_5$-$C_{21}$-Alkenyl, A $C_2$-$C_4$-Alkylen und M ein Metall- oder Ammoniumion bedeuten,
durch Neutralisation einer Acyloxialkansulfonsäure der Formel

$$R-\overset{\overset{\displaystyle O}{\|}}{C}-O-A-SO_3H$$

in nichtwäßrigem Medium, vorzugsweise in einer $C_2$-$C_4$-Carbonsäure, mit einem Metall- oder Ammoniumsalz, einer niedermolekularen, organischen Carbonsäure.

EP 0 327 926 A1

## Verfahren zur Herstellung von Salzen von Acyloxialkansulfonsäuren

Acyloxialkansulfonsäuren und deren Salze sind seit langem bekannte Verbindungen mit oberflächenaktiven Eigenschaften, die u.a. als Grundstoff zur Herstellung von Syndet-Seifen Verwendung finden.

Zu ihrer Darstellung sind bereits eine Reihe von Verfahren untersucht worden (J. Am. Oils Chem. Soc. 48, 657 (1971)). In US 1,881,172 und US 2,821,535 ist die Umsetzung von Na-Isethionat mit Fettsäurechloriden beschrieben. Diese Reaktion erfordert den Einsatz von völlig trockenem und feingepulvertem Na-Isethionat; zudem ist die Herstellung der Fettsäurehalogenide aus Fettsäure und Phosphortrichlorid zeit- und kostenintensiv und mit einem Zwangsanfall von Phosphoriger Säure verbunden. Bei Verwendung von undestilliertem Säurechlorid ist das Endprodukt meist durch phosphorhaltige Verbindungen verunreinigt, was zu geruchlichen Problemen führen kann. Die Direktkondensation von Na-Isethionat mit Fettsäuren erfordert Reaktionstemperaturen von 220 bis 250°C. Zur Vervollständigung dieser Reaktion muß meist ein Überschuß an Fettsäure eingesetzt werden. Die Reaktion kann ohne Katalysator (DE 1,121,045) sowie in Gegenwart von Zinkoxid (US 3,320,292) oder Zinksalzen organischer Säuren (US 3,004,049) durchgeführt werden. Bei Verwendung von gasförmigem Chlorwasserstoff als Katalysator ist eine Direktveresterung bei 180°C (US 3,167,570), bei wäßriger Salzsäure bei 130°C (DE 3706 232) möglich.

Nach US 3,151,136 gelingt die Veresterung von Isethionsäure mit Fettsäure bei 130°C. Diese Reaktion bietet eine Reihe von Vorteilen. Neben milden Reaktionsbedingungen ist es möglich, die Reaktion kontinuierlich zu führen und durch Neutralisation mit verschiedenen Basen zu Metall- oder Ammoniumsalzen von Acyloxialkansulfonsäuren zu gelangen, die auf anderen Wegen nur schwer herstellbar sind.

Wie in US 3,151,136 angegeben, bereitet jedoch die Neutralisation des Kondensationsproduktes erhebliche Schwierigkeiten und ist mit Natronlauge nur in Gegenwart von Natriumsilikaten möglich, die aber in vielen Anwendungsgebieten der Acylisethionate unerwünscht sind. Ohne diese Zusätze findet bei der Neutralisation in erheblichem Maße eine Esterspaltung statt. Durch Einsatz von Metallcarbonaten kann die Neutralisation zwar nahezu ohne Esterhydrolyse durchgeführt werden, jedoch verbietet das starke Schäumen der Reaktion eine großtechnische Anwendung.

Aufgabe der vorliegenden Erfindung ist es daher, ein auch im großtechnischen Maßstab leicht durchführbares Verfahren zur Herstellung von Metallsalzen von Acyloxialkansulfonsäuren aufzuzeigen, bei dem die Neutralisation der Acyloxialkansulfonsäure keine Schwierigkeiten bereitet.

Es wurde nun gefunden, daß die Neutralisation von Acyloxialkansulfonsäuren in nichtwäßrigem Medium mit Metall- oder Ammoniumsalzen von niedermolekularen organischen Carbonsäuren problemlos durchführbar und das Neutralisationsprodukt auf einfache Weise isolierbar ist.

Gegenstand der Erfindung ist daher ein Verfahren zur Herstellung von Salzen von Acyloxialkansulfonsäuren der Formel

$$R-\overset{O}{\overset{\|}{C}}-O-A-SO_3M$$

wobei R $C_5$-$C_{21}$-Alkyl oder $C_5$-$C_{21}$-Alkenyl, A $C_2$-$C_4$-Alkylen und M ein Metall- oder Ammonium-Ion bedeuten, dadurch gekennzeichnet, daß eine Acyloxialkansulfonsäure der allgemeinen Formel

$$R-\overset{O}{\overset{\|}{C}}-O-A-SO_3H$$

wobei R und A die oben angegebene Bedeutung haben, in nichtwäßrigem Medium, vorzugsweise einer niedermolekularen organischen Carbonsäure, mit einem Metall- oder Ammoniumsalz einer niedermolekularen organischen Carbonsäure bei 10 bis 100°C umgesetzt wird. Das Neutralisationsprodukt ist in dem nichtwäßrigen Medium nur wenig löslich und wird durch Filtration oder Abzentrifugieren gewonnen.

Die Acyloxialkansulfonsäure kann auf verschiedenen Wegen hergestellt werden, so z.B. durch Direktkondensation von Fettsäuren mit Hydroxialkansulfonsäuren, insbesondere Isethionsäure wie in US 3,151,136 beschreiben. Weiterhin können die Acyloxialkansulfonsäuren auch durch Umsetzung von Hydroxialkansulfonsäure mit Fettsäurederivaten erhalten werden. So gelingt die Reaktion von Isethionsäure mit Fettsäurehalogeniden, insbesondere -chloriden bei 60 bis 90°C, mit Fettsäureanhydriden bei 80 bis 100°C und mit Fettsäuremethylestern bei 100 bis 140°C. In allen Fällen erhält man die Acyloxialkansulfonsäure in hohen Ausbeuten in Form einer rotbraunen Masse mit Gehalten an Wirksubstanz von über 75 % (Ausnahme: Umsetzung mit Anhydrid, da das Reaktionsprodukt ein Mol Fettsäure enthält). Zur Neutralisation der Acyloxialkansulfonsäure wird diese in einem nichtwäßrigem Medium, vorzugsweise einer niedermolekularen organischen Carbonsäure mit 2 bis 4 Kohlenstoffatomen wie Essigsäure oder Propionsäure bei 10 bis 100°C, vorzugsweise 40 bis 80°C, gelöst und durch Zugabe eines Metall- oder Ammoniumsalzes einer niedermolekularen organischen Carbonsäure mit 2 bis 4 Kohlenstoffatomen wie Natrium-, Kalium-, Ammonium-, Calcium-, Zink-, oder Magnesiumacetat oder -propionat bei 10 bis 100° C, vorzugsweise 20 bis 80° C, neutralisiert. Das Metall- oder Ammoniumsalz kann dabei in fester Form oder als Lösung in einer niedermolekularen organischen Säure zugegeben werden. Die zunächst rotbraune Lösung hellt während der Neutralisation auf und das Neutralisationsprodukt kristallisiert in Form weißer Kristalle aus. Das Reaktionsgemisch wird nach erfolgter Neutralisation auf 0 bis 40° C, vorzugsweise 0 bis 20° C, abgekühlt und das Acyloxialkansulfonat durch Filtration oder Abzentrifugieren in reiner Form gewonnen und nach herkömmlichen Methoden getrocknet. Die in der Mutterlauge enthaltenen Wertstoffe wie Fettsäuren, Hydroxialkansulfonsäure, Hydroxialkansulfonat oder gelöstes Neutralisationsprodukt können nach quantitativer Abtrennung des Lösemittels ohne weitere Reinigung erneut der Kondensation zugeführt werden.

Das Gewichtsverhältnis der zu neutralisierenden Acyloxialkansulfonsäure zu nichtwäßrigem Lösemittel beträgt 1 : 1 bis 10, vorzugsweise 1 : 1 bis 5. Das Molverhältnis von Acyloxialkansulfonsäure zu Metall- oder Ammoniumsalz einer niedermolekularen organischen Säure beträgt 1 : 0,5 bis 1,5, vorzugsweise 1 : 0,7 bis 1,2.

In den folgenden Beispielen verhalten sich Gew.-Teile zu Vol.-Teilen wie kg zu dm³, %-Angaben beziehen sich - wenn nichts anderes angegeben - immer auf das Gewicht. Unter dem Begriff WS-Gehalt ist in den nachfolgendne Beispielen der Anteil an Wirksubstanz im Produkt zu verstehen, der durch 2-Phasen-Titration nach EPTON (Nature 160, 759 (1947)) bestimmt wird.

**Beispiel 1**

244,2 Teile Kokosfettsäure werden auf 110° C erwärmt, es wird Wasserstrahlvakuum angelegt und 134 Teile Isethionsäure (94 proz.) werden innerhalb von 30 min zugetropft, wobei das Reaktionswasser abdestilliert. Es wird je 30 min bei 110 bis 120° C und 135° C nachgerührt (WS-Gehalt 80,6 %). Das Reaktionsgemisch wird auf 75° C abgekühlt, 600 ml Essigsäure werden zugegeben und verrührt. Bei 40° C beginnend werden 82 Teile Natriumacetat eingetragen und 1 h nachgerührt. Acyloxialkansulfonat-Na fällt in Form weißer Kristalle aus. Nach Abkühlen auf 10° C wird das Produkt abgesaugt, mit wenig Essigsäure gewaschen und über Nacht im Trockenschrank getrocknet.
Ausbeute: 330 Teile Acyloxialkansulfonat-Na als weißes Pulver mit einem WS-Gehalt von 90,5 %

**Beispiel 2**

244,2 Teile gehärtete Kokosfettsäure (C₁₂-C₁₈) werden auf 110° C erwärmt. Nach Anlegen eines Wasserstrahlvakuums werden bei 110 bis 120° C 252 Teile technische Isethionsäure-Lösung (50 proz.) innerhalb 1 h zugetropft, während das Wasser kontinuierlich abdestilliert wird. Nach beendeter Zugabe wird 1 h bei 130 bis 140° C nachgerührt. Man erhält eine Acyloxialkansulfonsäure mit einem WS-Gehalt von 83,5 %. Die braune Masse wird in 600 ml Essigsäure gelöst und durch Zugabe von 82 Teilen Natriumacetat neutralisiert. Das Produkt wird in Form weißer Kristalle isoliert, die 12 h im Vakuumtrockenschrank getrocknet werden.

Man erhält 325 Teile Acyloxialkansulfonat-Na in Form eines weißen, geruchlosen Pulvers mit einem WS-Gehalt von 92,3 %.

**Beispiel 3**

240 Teile Laurinsäuremethylester und 126 Teile Isethionsäure werden unter Vakuum (20 mm) 1 h auf 130 bis 135° C erhitzt. Währenddessen wird der gebildete Alkohol kontinuierlich abdestilliert. Man erhält eine rohe Lauroyloxiethansulfonsäure mit einem WS-Gehalt von 77,2 %. Durch Neutralisation mit Natriumacetat in Essigsäure bei 50° C und Isolierung des Lauroyloxiethansulfonat-Na erhält man ein weißes Pulver mit einem WS-Gehalt von 85,7 %.

**Ansprüche**

1. Verfahren zur Herstellung von Acyloxialkansulfonaten der Formel

$$R-\overset{O}{\overset{\|}{C}}-O-A-SO_3M$$

wobei R C₅-C₂₁-Alkyl oder C₅-C₂₁-Alkenyl, A C₂-C₄-Alkylen und M ein Metall- oder Ammoniumion bedeuten,
dadurch gekennzeichnet, daß man eine Acyloxialkansulfonsäure der allgemeinen Formel

$$R-\overset{O}{\overset{\|}{C}}-O-A-SO_3H$$

wobei R und A die oben angegebenen Bedeutungen haben, in nichtwäßrigem Medium mit dem Metall- oder Ammoniumsalz einer niedermolekularen organischen Carbonsäure umsetzt.

2. Verfahren nach Anspruch 1, dadurch gekennzeichnet, daß das nichtwäßrige Medium eine niedermolekulare organische Carbonsäure ist.

3. Verfahren nach Anspruch 1 und 2, dadurch gekennzeichnet, daß das nichtwäßrige Medium Essigsäure ist.

4. Verfahren nach Anspruch 3, dadurch gekennzeichnet, daß die Reaktion bei 10 bis 100° C durchgeführt wird.

5. Verfahren nach Ansprüchen 1-4, dadurch gekennzeichnet, daß die Acyloxialkansulfonsäure durch Umsetzung einer Hydroxialkansulfonsäure mit Carbonsäuren, Carbonsäurehalogeniden, Carbonsäureanhydriden oder Carbonsäureestern erhalten wird.

| | Europäisches Patentamt | EUROPÄISCHER RECHERCHENBERICHT | Nummer der Anmeldung |
|---|---|---|---|

EP 89 10 1617

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 227 194 (UNILEVER NV) <br> * Spalte 6, Absatz 3, Zeilen 9-12; Spalte 10; Ansprüche 1(3), 2-4 * <br> --- | 1-5 | C 07 C 143/10 <br> C 07 C 139/14 |
| A | HOUBEN-WEYL,"Methoden der Organischen Chemie", Band 9, Schwefel-, Selen,- Tellur-Verbindungen, 1955, Georg Thieme Verlag, Stuttgart, DE <br> * Seite 441, Absatz 4 * <br> --- | 1-5 | |
| D,A | US-A-3 151 136 (E. KOCKZOROWSKI) <br> * Ansprüche * <br> ----- | 1-5 | |

RECHERCHIERTE SACHGEBIETE (Int. Cl.4)

C 07 C 143/00
C 07 C 139/00

Der vorliegende Recherchenbericht wurde für alle Patentansprüche erstellt

| Recherchenort | Abschlußdatum der Recherche | Prüfer |
|---|---|---|
| DEN HAAG | 12-04-1989 | ZAROKOSTAS K. |

EPO FORM 1503 03.82 (P0403)

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

................................................................
& : Mitglied der gleichen Patentfamilie, übereinstimmendes Dokument